# EUROPEAN PATENT APPLICATION

(11) **EP 2 759 315 A1**
(43) Date of publication of application: **30.07.2014**
(21) Application number: 13425016.6
(22) Date of filing: 25.01.2013
(51) Int. Cl.: A61N 2/00, A61B 5/04, A61B 5/0484

(54) **Brain Synchronisation using Magnetic Stimulation**

(71) Applicant: Universita'del Salento, 73100 Lecce (IT)
(72) Inventor: Invitto, Sara, 73100 San Cesario di Lecce (IT)
(74) Representative: Bruni, Giovanni

(57) **Abstract**

Electromagnetic device for stimulating a particular encephalic area comprising at least a current generator and characterized in that it is provided with at least a coil of circular shape for stimulating a portion of the brain lobes in a localized way and in that it generates an electric power between 0 and 50 Watt at a frequency between 3 and 20 Hz adjustable in continuum to determine a brain synchronization effect in response to an applied magnetic field.

## Description

The present invention relates to a new electromagnetic device, a frequency generator, able to generate regular and long term low frequency fields to determine if it is provided a brain synchronization effect of neuronal clusters in response to an outer electric and magnetic field. Neurons work through electric potentials given by charge changes between the membrane inside and outside. These potentials can be recorded by means of brain imaging techniques and by means of electroencephalographic technique. Said potentials can be modified also by means of brain stimulation processes.

In scientific literature there are known slow waves, better known by the acronym SWS meaning in English Slow Wave Sleep, used to describe the 3rd and the 4th stage of the sleep, which are the deepest stages. The electro-encephalic features which can be visualized through an encephalogram tracing (EEG) provide these waves as great delta waves with amplitude greater than 75 microvolt and frequency of 2 Hz. The stage 3 is defined by Rechtschaffen and Kales as the stage in which the delta waves are about 20-50% of an epoch of 30 seconds of the EEG (generally equal to 8-13 delta waves). Specifically, the waking attentive state causes variations which can be highlighted as electric signal with irregular amplitude and frequency (beta rhythm: 14-30 Hz with an amplitude of 1-20 millivolt) or a frequency and amplitude slightly more regulated in function of relaxed waking stages (alpha: 8-13 Hz, amplitude 10-200 millivolt). The electro-encephalic activation can be both exogenous (perceptive stimuli which cause activation responses) and endogenous when there is an electrophysiological modulation guided by vegetative and brain stem channels. A particular proof of the brain stem and vegetative endogenous alteration which causes an electro-encephalic synchronization in frequency and amplitude is the slow wave sleep (SWS). As yet stated, SWS is an active phenomenon, provided physiologically by the activation of serotonergic neurons of the system of the mesencephalic raphe nuclei. The slow waves observed in the cortical EEG are generated through thalamocortical neuronal connections; in these neurons this rhythm is generated by the spontaneous "slow oscillation" of the bi-stable membrane potential, a property of these neurons due to an electro-physiological component known as "I t window". The I t Window is due to an overlap underneath activation/inactivation curves if plotted through T-type calcium channels (inward current).

Unlike REM which is asynchronous, in SWS there is an increasing regularization and synchronization of the brain areas on a theta frequency of 3-7 Hz with an amplitude of 5-100 millivolt, until, in determined stages, the delta 0,5-3Hz with an amplitude of 20-200 millivolt is reached.

The inventive device is theoretically based on physical and electric resonance systems: each system oscillates freely with a proper frequency which depends on its geometric, physical and chemical features. The resonance phenomenon is observed when the period (frequency) of the system, forced to follow the motion imposed by an outer periodical force, is equal to a period of this latter. When such a condition occurs, the system receives energy from outside continually. This should occur also for the brain system, which besides having an endogenous activity, always responds to exogenous activations. A sensorial or perceptive event produces mainly two kinds of variations: a re-alignment of the stage of the brain rhythms and a power increase at some frequencies. The responses produced by the sensorial stimulation are defined Evoked Potential or ERP. Evoked Response Potential are observed in the tracing as variations both through EEG (i.e. a measure of the extracellular current flow which is generated by the sum of the activities of an high quantity of neurons); the surface potentials are mainly the result of the activity of the cortical pyramidal neurons arranged at the cortical area underneath the electrode, and through magneto-electroencephalografy MEG. MEG, more than EEG, can measure variations in the magnetic field produced by the intracellular currents of the cortical pyramidal neurons: it is based on ultra-sensible sensors called SQUID (Superconducting Quantum Interference Device) which produce both an alignment of the signal stage and a power increase or inhibition. To analyze the endogenous/exogenous activation level it is needed to take the direct problem in account, which considers the distribution of the electric potential on the cranial vault for a given electric source arranged in a conductive means or conductive volume. The electromagnetic source is represented here by the activated cortical area and the conductive volume is represented by the cranial vault. The equations regulating these measurements are the Maxwell equations and provide the electric and magnetic field values generated by any kind of source, given the properties of the same source (intensity, position etc...) and of the conductive volume (conductivity etc...). Recent studies have highlighted that electromagnetic devices, whose magnetic circuit has a maximum induction equal to two tesla, can generate a variable magnetic field and then an induced brain current.

A magnetic stimulation device was produced in 2002 and is called TMS (Transcranial Magnetic Stimulation). The TMS causes an impulse of variable and repeated intensity which causes an inhibitory or excitatory response in the area portion perpendicular to the current supply. Given the intensity of the magnetic field, the stimulation is activated for 1-2 seconds with an interval not lower than 3 seconds. The stimulation effect lasts for about 200 milliseconds.

Moreover it is important to consider the presence of mirror neurons, present in the motor, pre-motor cortex, and partially of the dorsal visual pathway, which are activated in function of an action resonance of another subject. On the basis of this recent finding, at phylogenetic level we should be resonant to the biological environmental action and activation. In cognitive and social field this effect occurs on other levels called "background effect".

On the basis of these data, aim of the present invention is to provide, by means of an *ad hoc* device, low, repeated frequencies magnetic fields in order to determine if it is provided a brain synchronization effect in response to an outer electric/magnetic field.

In particular, it is advantageous to use the electro-encephalic frequencies present in SWS, given their particular regularity and non casualness (the frequencies of the Beta rhythm, of waking, are irregular and difficult to be reproduced).

These and other advantages will be highlighted in the following detailed description which will refer specifically to figures 1-3, in which it is shown a preferred and absolutely not limiting embodiment of the present invention. In particular:
fig. 1 shows a scheme of the magnetic device according to the invention;
fig. 2 shows an application scheme of the magnetic device according to a second embodiment (side section of a stimulation helmet);
fig. 3 shows an application scheme of the magnetic device according to another embodiment (side section of a stimulation cap).

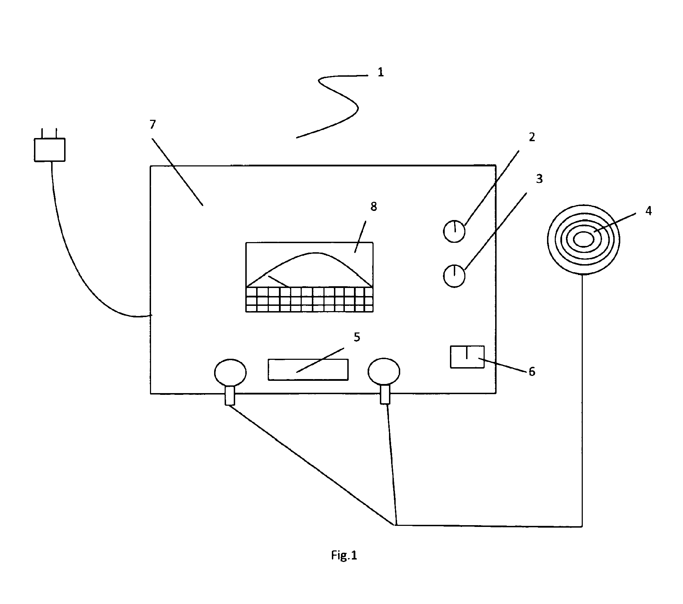

As it is shown in figure 1, the new electromagnetic device 1, object of the present invention, according to a preferred embodiment of the invention, comprises at least a coil 4 of circular shape so that it can stimulate a portion of the brain lobes in a localized way. Said electromagnetic device is made up of a current generator 7 comprising an electromagnet 5 and having in the frontal portion an on/off switch 6, an intensity 2 and frequency 3 regulation knob of the supplied current, and a display for reading the same current 8. Said electromagnet 5 is connected at one of its ends to a coil 4 which will be applied directly to the skull at the area intended to be stimulated.

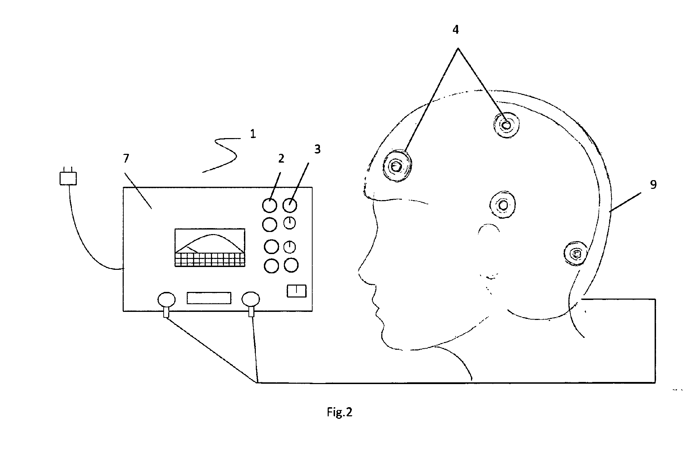

Figure 2 shows the device according to the invention in an alternative embodiment wherein there is provided a quantity of coils 4 equal to eight, arranged inside the helmet shaped support structure 9, each one being adjustable independently of each other for intensity 2 and frequency 3, through the corresponding regulation knobs provided on the current generator 7.

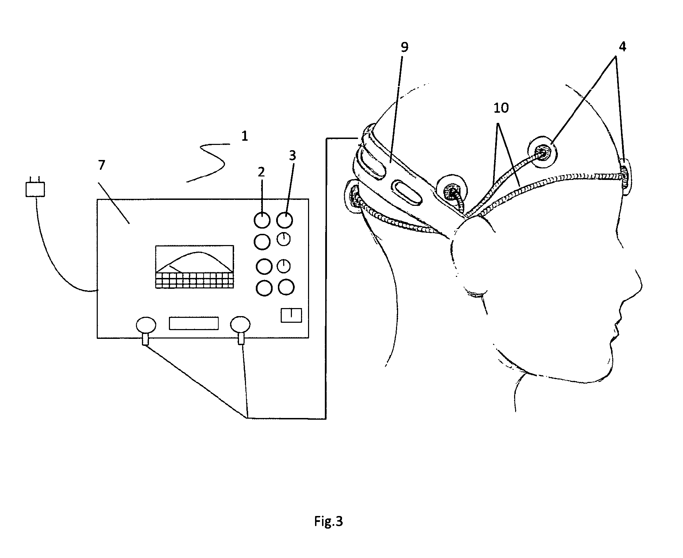

Another embodiment of the present invention, as shown in figure 3, is represented by the quantity of coils 4 equal to eight connected to a cap shaped support structure 9 with four arms 10 for hemisphere, each arm having a coil 4 at its end. Each coil 4 can be adjusted independently of each other for intensity 2 and frequency 3 through the corresponding regulation knobs provided on the current generator 7.

The experimentation model modulates the EEG frequency in function of the conductance/resistance of the cranial vault and can modulate EEG effects and the attention levels, after about 30 minutes of frequency supply.

The coils are arranged so that they are distributed each one for each brain lobe (parietal, frontal, occipital, temporal) and in bilateral way for a total of eight coils. Each coil can be activated singularly or in parallel or also all the coils can be activated synchronously.

The frequency intensity generated varies between 3 and 20 Hz and can be adjusted in continuum. The parameters to be observed are the physiological-like ones: 5 minutes at 20 Hz, 5 minutes at 15 Hz, 5 minutes at 10 Hz and 5 minutes at 5 Hz, for a maximum of 20/30 minutes in decreasing way and 20/30 minutes in increasing way. The electromagnetic field reproduced is of low entity so that it does not provide a cortical inhibitory effect, but simply induces a resonance of variations of frequencies.

The device object of the present invention is able to generate a power variable between 0 and 50 Watt (adjustable according to the test to be carried out), at a frequency between 3 and 20 Hz adjustable in continuum. Said device is made up of a generator comprising a quantity of two 3.15 A fuses and one 500 µA fuse provided with earthing plug.

An embodiment of the inventive device comprises a quantity of two turns with 3 cm in diameter and 10 cm in radius. Each turn can be connected to a frequency meter. In addition to the above cited intensity and frequency, the electromagnetic features of the device have an axial magnetic field variable between 0.2 Mt and 28 mT and a transverse magnetic field <0.1 mT.

The stimulation method of a particular encephalic area which uses the induction device object of the present invention, after the device is positioned on the cranial vault, is defined according to the following decreasing stimulation steps:
- first stimulation at 20 Hz for 5 minutes;
- second stimulation at 15 Hz for 5 minutes;
- third stimulation at 10 Hz for 5 minutes;
- fourth stimulation at 5 Hz for 5 minutes.

At this point, it is possible to carry out a brain synchronization level recording by means of EEG or MEG.

Usually, the stimulation is continued according to the additional increasing stimulation steps:
- stimulation at 5 Hz for 5 minutes;
- stimulation at 10 Hz for 5 minutes;
- stimulation at 15 Hz for 5 minutes;
- stimulation at 20 Hz for 5 minutes.

After the eight stimulation steps, the brain synchronization level is determined through EEG recording on the stimulated area, or through MEG recording.

By arranging one or more electrodes in the stimulated positions or the cap when the irradiation was on the four lobes of both the hemispheres, the synchronization effect on the low frequencies is recorded. The synchronization effect can occur also with lower stimulation levels (after the first four stimulations).

In the following there are described some examples relating to the measurements on ranges of the axial magnetic field according to the variation of the dimensions of the applied coil:

### Example 1

Coil 3 cm in diameter
16 Watt-3 Hz: 15 millitesla
33 Watt-3 Hz: 20 millitesla
50 Watt-3 Hz: 28 millitesla

### Example 2

Coil 3 cm in diameter
16 Watt-11,5 Hz: 14 millitesla
33 Watt-11,5 Hz: 22 millitesla
50 Watt-11,5 Hz: 25,5 millitesla

### Example 3

Coil 3 cm in diameter
16 Watt-20 Hz: 18 millitesla
33 Watt-20 Hz: 21 millitesla
50 Watt-20 Hz: 22 millitesla

### Example 4

Coil 10 cm in diameter
16 Watt-3 Hz: 0,2 millitesla
33 Watt-3 Hz: 0,4 millitesla
50 Watt-3 Hz: 0,7 millitesla

### Example 5

Coil 10 cm in diameter
16 Watt-11,5 Hz: 0,2 millitesla
33 Watt-11,5 Hz: 0,4 millitesla 50 Watt-11,5 Hz: 0,7 millitesla

### Example 6

Coil 10 cm in diameter
16 Watt-20 Hz: 0,2 millitesla
33 Watt-20 Hz: 0,4 millitesla
50 Watt-20 Hz: 0,7 millitesla

The above described data indicate the presence, by exerting a slight power (16 Watt and 33 Watt) of a sound which should be shielded because it can cause interference and can become another variable which intervenes in the effect of regular frequencies induction.

## Claims

1. Electromagnetic device (1) for stimulating a particular encephalic area comprising at least a current generator (7) and **characterized in that** it is provided with at least a coil (4) of circular shape for stimulating a portion of the brain lobes in a localized way and **in that** it generates an electric power between 0 and 50 Watt at a frequency between 3 and 20 Hz adjustable in continuum to determine a brain synchronization effect in response to an outer electric or magnetic field.

2. Device according to claim 1, **characterized in that** said coil (4) is positioned at a brain lobe.

3. Device according to claim 1 or 2, **characterized in that** it has a quantity of coils (4) equal to eight.

4. Device according to claim 3, **characterized in that** each coil (4) can be adjusted independently of each other in intensity and frequency by means of knobs (2, 3) which are positioned on the current generator (7).

5. Device according to claim 3, **characterized in that** the coils (4) are arranged inside a supporting structure (9).

6. Device according to claim 5, **characterized in that** said supporting structure (9) is a helmet.

7. Device according to claim 5, **characterized in that** said supporting structure (9) is a cap with four arms (10) for hemisphere, each arm having a coil (4) at its end.

8. Device according to any one of claims 1 to 7, **characterized in that** the value of the electric power is 50 Watt.

9. Method for stimulating a particular encephalic area using an electromagnetic device according to any one of claims 1 to 7, **characterized by** the following steps:
- positioning the device on the cranial vault;
- first stimulation at 20 Hz for 5 minutes;
- second stimulation at 15 Hz for 5 minutes;
- third stimulation at 10 Hz for 5 minutes;
- fourth stimulation at 5 Hz for 5 minutes.
- recording the brain synchronization level by means of EEG or MEG.

10. Method according to claim 4, **characterized by** the following additional steps:
- stimulation at 5 Hz for 5 minutes;
- stimulation at 10 Hz for 5 minutes;
- stimulation at 15 Hz for 5 minutes;
- stimulation at 20 Hz for 5 minutes.
- recording the brain synchronization level by means of EEG or MEG.

11. Method according to claim 7 and 8, **characterized in that** the maximum period of stimulation is in a range between 20 and 30 minutes.
